(19) Europäisches Patentamt
European Patent Office
Office européen des brevets

(11) Veröffentlichungsnummer: **0 154 178 B1**

# (12) EUROPÄISCHE PATENTSCHRIFT

(45) Veröffentlichungstag der Patentschrift: **07.10.87**

(51) Int. Cl.⁴: **C 07 D 401/06**, A 01 N 43/54, A 01 N 43/50

(21) Anmeldenummer: **85101173.4**

(22) Anmeldetag: **05.02.85**

(54) **Nitromethylen-Derivate, Verfahren zur ihrer Herstellung sowie insektizide, mitizide und nematizide Mittel.**

(30) Priorität: **16.02.84 JP 26020/84**

(43) Veröffentlichungstag der Anmeldung: **11.09.85 Patentblatt 85/37**

(45) Bekanntmachung des Hinweises auf die Patenterteilung: **07.10.87 Patentblatt 87/41**

(84) Benannte Vertragsstaaten: **AT BE CH DE FR GB IT LI NL**

(56) Entgegenhaltungen:
**DE - A - 2 514 402**
**DE - A - 2 732 660**
**GB - A - 2 014 147**
**US - A - 3 971 774**
**US - A - 4 002 765**

**CHEMICAL ABSTRACTS, Band 90, No. 10, 5. März 1979, Columbus, Ohio, USA RASMUSSEN, C.A. H,; VAN DER PLAS, H.C.; GROTENHUIS, P.; KOUDIJS, A. "Pyrimidines. Part LXX. investigations into the cine-amination of 4-substituted-5-bromopyrimidines by potassium amide in liquid ammonia." Seite 522, Spalte 1, Zusammenfassung-Nr. 86 292q**

**Die Akte enthält technische Angaben, die nach dem Eingang der Anmeldung eingereicht wurden und die nicht in dieser Patentschrift enthalten sind.**

(73) Patentinhaber: **NIHON TOKUSHU NOYAKU SEIZO K.K., No.4, 2-Chome, Nihonbashi Honcho, Chuo-ku Tokyo 103 (JP)**

(72) Erfinder: **Shiokawa, Kozo, 210-6, Shukugawara Tama-ku, Kawasaki-shi Kanagawa-ken (JP)**
Erfinder: **Tsuboi, Shinichi, 3-26-1, Hirayama, Hino-shi Tokyo (JP)**
Erfinder: **Kagabu, Shinzo, 432-131-105, Terada-machi, Hachioji-shi Tokyo (JP)**
Erfinder: **Koichi, Moriya, 39-15, Namiki-cho, Hachioji-shi Tokyo (JP)**

(74) Vertreter: **Ernst, Hilmar, Dr. et al, Bayer AG Konzernverwaltung RP Patentabteilung, D-5090 Leverkusen, Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 154 178 B1

ACTORUM AG

**Beschreibung**

Die vorliegende Erfindung betrifft neue Nitromethylen-Derivate, Verfahren zu ihrer Herstellung sowie insektizide, mitizide und nematizide Mittel.

Insbesondere betrifft die vorliegende Erfindung neue Nitromethylen-Derivate der nachstehenden allgemeinen Formel (I)

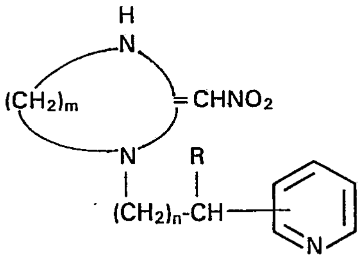

(I),

in der

R ein Wasserstoff-Atom, Methyl, Ethyl, Propyl, i-Propyl, n-Butyl, i-Butyl, sec.Butyl oder tert.Butyl bezeichnet,

m für 2, 3 oder 4 steht und

n für 0, 1, 2 oder 3 steht.

Die Nitromethylen-Derivate der Formel (I) gemäss der vorliegenden Erfindung können mittels des folgenden allgemeinen Verfahrens i) hergestellt werden, auf das sich die vorliegende Erfindung ebenfalls erstreckt.

Ein Verfahren zur Herstellung der Nitromethylen-Derivate der allgemeinen Formel (I) umfasst die Reaktion einer Verbindung der allgemeinen Formel

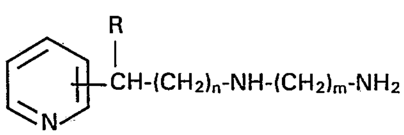

(II),

in der R, m und n die im vorstehenden angegebenen Bedeutungen haben, mit einer Verbindung der allgemeinen Formel

R'-S
 >C = $CHNO_2$ (III),
R'-S

in der jedes R' eine niedere Alkyl-Gruppe bezeichnet oder die beiden R'-Gruppen zusammen eine niedere Alkylen-Gruppe mit wenigstens 2 Kohlenstoff-Atomen bezeichnet und mit den ihnen benachbarten Schwefel-Atomen einen Ring bilden können.

Die vorliegende Erfindung betrifft weiterhin insektizide, mitizide und nematizide Mittel, die Nitromethylen-Derivate der Formel (I) als Wirkstoffe enthalten.

Die vor dem Anmeldezeitpunkt der vorliegenden Anmeldung bekannte DE-OS 2 514 402 offenbart, dass 2-Nitromethylen-imidazolin-Derivate und 2-Nitromethylen-hexahydropyrimidin-Derivate der nachstehenden allgemeinen Formel

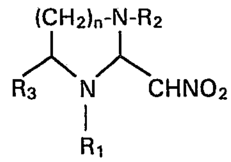

insektizide Aktivität zeigen. Die vorstehende allgemeine Formel umfasst Fälle mit n = 2, $R_1$ = Phenyl-($C_1$-$C_2$)-alkyl-Gruppe und $R_2$ = $R_3$ = Wasserstoff, und die Beschreibung der DE-OS 2 514 402 offenbart eine Verbindung der nachstehenden Formel:

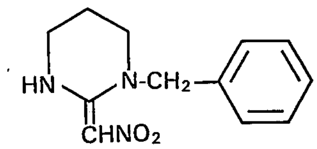

(A-1)

Aus der DE-OS 2 732 660 ist bekannt, dass 1-substituiertes Benzyl-2-nitromethylen-imidazolidin-Derivate der nachstehenden allgemeinen Formel

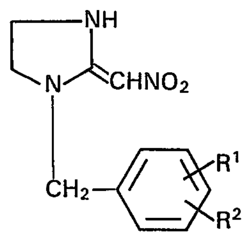

insektizide Aktivität besitzen. Die Beschreibung dieser Offenlegungsschrift offenbart eine Verbindung der nachstehenden Formel:

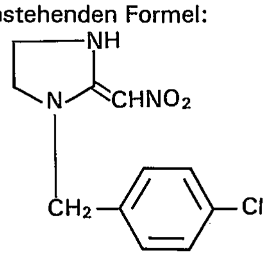

(B-1)

Zur Schaffung neuer wertvoller Verbindungen mit biologischer Aktivität werden nun neue Nitromethylen-tetrahydropyrimidin-Verbindungen und Nitromethylen-imidazolidin-Verbindungen der allgemeinen Formel (I) bereitgestellt, welche eine hervorragende Bekämpfung gegenüber schädlichen Insekten, Milben und Nematoden besitzen.

Die Verbindungen der vorliegenden Erfindung sind gekennzeichnet durch die Tatsache, dass in ihrer chemischen Struktur 2-Nitromethylen-tetrahydropyrimidin, 2-Nitromethylen-imidazolidin oder 2-Nitromethylen-hexahydro-1,3-diazepin ein Grundskelett bildet und, wie aus der allgemeinen Formel (I) deutlich hervorgeht, eine Pyridylalkyl-Gruppe an dem Stickstoff-Atom in der 1-Stellung des genannten Hetero-Rings substituiert ist. Überraschenderweise wurde gefunden, dass eine Korrelation besteht zwischen dieser chemischen Struktur und der Ausübung hoher Aktivität (Bekämpfungswirksamkeit) durch die Verbindungen der Formel (I). Ausserdem wurde gefunden, dass eine besonders überlegene Korrelation nachgewiesen wird, wenn die Pyridylalkyl-Gruppe speziell eine 3-Pyridylmethyl-Gruppe oder 4-Pyridylmethyl-Gruppe ist.

Es wurde ausserdem gefunden, dass die Verbindungen der vorliegenden Erfindung bei niedrigen Dosierungen eine besonders hervorragende Bekämpfungswirkung besitzen, die diejenige der Verbindungen der Formeln (A-1) und (B-1), die in den oben genannten deutschen Offenlegungsschriften beschrieben sind und den Verbindungen der vorliegenden Erfindung am ähnlichsten sind, weit übertrifft, und dass die Verbindungen der vorliegenden Erfindung eine ausgeprägte Bekämpfungswirkung gegen Schadinsekten zeigen, die gegenüber Insektiziden vom Typ organischer Phosphate und vom Carbamat-Typ aufgrund langdauernden Einsatzes derselben eine Resistenz entwickelt haben, insbesondere gegen saugende Insekten, wie z.B. Insekten der Ordnung Hemiptera wie Aphiden, Laternenträger und Zikaden.

Die erfindungsgemässen Wirkstoffe zeigen eine Bekämpfungswirkung gegen schädliche Insekten, Milben oder Zecken und Nematoden, ohne dass Phytotoxizität gegenüber Kulturpflanzen auftritt. Des weiteren können die Verbindungen der vorliegenden Erfindung zur Bekämpfung und Ausrottung einer breiten Palette von Schädlingen eingesetzt werden, darunter saugenden Insekten, beissenden Insekten und anderen Pflanzenparasiten, Schädlingen von Lagergetreide und gesundheitsgefährdenden Schädlingen.

Beispiele für diese Schädlinge sind nachstehend aufgeführt:

*Insekten der Ordnung Coleoptera*

Callosobruchus chinensis,
Sitophilus zeamais,
Tribolium castaneum,
Epilachna vigintioctomaculata,
Agriotes fuscicollis,
Anomala rufocuprea,
Leptinotarsa decemlineata,
Diabrotica spp.,
Monochamus alternatus,
Lissorhoptus oryzophilus und
Lyctus brunneus.

*Insekten der Ordnung Lepidoptera*

Lymantria dispar,
Malacosoma neustria,
Pieris rapae,
Spodoptera litura,
Mamestra brassicae,
Chilo suppressalis,
Pyrausta nubilalis,
Ephestia cautella,
Adoxophyes orana,
Carpocapsa pomonella,
Agrotis fucosa,
Galleria mellonella
Plutella maculipennis und
Phyllocnistis citrella.

*Insekten der Ordnung Hemiptera*

Nephotettix cincticeps,
Nilaparvata lugens,
Pseudococcus comstocki,
Unaspis yanonensis,
Myzus persicae,
Aphis pomi,
Aphis gossypii,
Rhopalosiphum pseudobrassicas,
Stephanitis nashi,
Nazara spp.,
Cimex lectularius,
Trialeurodes vaporariorum und
Psylla spp.

*Insekten der Ordnung Orthoptera*

Blatella germanica,
Periplaneta americana,
Gryllotalpa africana und
Locusta migratoria migratoriodes.

*Insekten der Ordnung Isoptera*

Deucotermes speratus und
Coptotermes formosanus.

*Insekten der Ordnung Diptera*

Musca domestica,
Aedes aegypti,
Hylemia platura,
Culex pipiens,
Anopheles sinensis und
Culex tritaeniorhynchus.

*Milben*

Tetranychus telarius,
Panonychus citri,
Aculus pelekassi und
Torronomus spp.

*Nematoden*

Meloidogyne incognita,
Bursaphelenchus lignicolus Mamiya et Kiyohara,
Aphelenchoides besseyi,
Heterodera glycines und
Pratylenchus spp.

Auf dem Gebiet der Veterinärmedizin sind die neuen Verbindungen gemäss der vorliegenden Erfindung wirksam gegen verschiedene schädliche Tierparasiten (Endo- und Ektoparasiten) wie Zecken, Insekten und Würmer. Beispiele für solche Tierparasiten sind nachstehend angegeben.

*Zecken*

Oranithodoros spp.,
Ixodes spp. und
Boophilus spp.

*Insekten*

Gastrophilus spp.,
Stomoxys spp.,
Trichodectes spp.,
Rhodnius spp. und
Ctenocephalidex canis.

Substanzen, die eine pestizide Aktivität gegenüber all diesen Schädlingen aufweisen, werden in der vorliegenden Anmeldung gelegentlich einfach als «Insektizide» bezeichnet.

Die Nitromethylen-Derivate der allgemeinen Formel (I) gemäss der vorliegenden Erfindung können in einfacher Weise hergestellt werden, beispielsweise mit Hilfe des folgenden Verfahrens i).

*Verfahren i)*

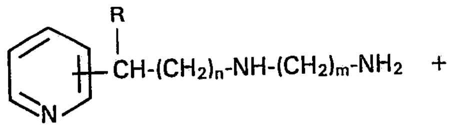

(II)

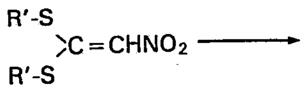

(III)

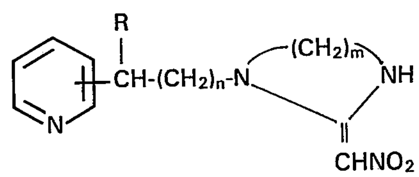

(I)

(In den Formeln haben R, m, n und R' die im vorstehenden angegebenen Bedeutungen.)

In dem vorstehenden Reaktionsschema bezeichnet R ein Wasserstoff-Atom, Methyl, Ethyl, Propyl, Isopropyl, n-Butyl, i-Butyl, sec.Butyl oder tert.Butyl. m bezeichnet 2,3 oder 4, und n bezeichnet 0, 1, 2 oder 3. R' bezeichnet eine niedere Alkylgruppe, oder die beiden Gruppen R' zusammen bezeichnen eine niedere Alkylen-Gruppe mit wenigstens 2 Kohlenstoff-Atomen.

Spezielle Beispiele für die niedere Alkyl-Gruppe sind die gleichen wie diejenigen, die oben für R angegeben sind. Die beiden Gruppen R' zusammen bezeichnen eine niedere Alkylen-Gruppe mit wenigstens 2 Kohlenstoff-Atomen und können zusammen mit den ihnen benachbarten Schwefel-Atomen einen Ring bilden. Eine Ethylen-Gruppe ist als Beispiel für eine solche Alkylen-Gruppe zu nennen.

In dem durch das obige Reaktionsschema dargestellten Verfahren zur Herstellung der Verbindung der allgemeinen Formel (I) zählen zu speziellen Beispielen für die Ausgangs-Verbindung der allgemeinen Formel (II)

N-(2-Pyridylmethyl)ethylendiamin,
N-(3-Pyridylmethyl)ethylendiamin,
N-(4-Pyridylmethyl)ethylendiamin,
N-[2-(4-Pyridyl)ethyl]ethylendiamin,
N-(2-Pyridylmethyl)trimethylendiamin,
N-[2-(2-Pyridyl)ethyl]trimethylendiamin,
N-(3-Pyridylmethyl)trimethylendiamin,
N-[1-(3-Pyridyl)ethyl]trimethylendiamin,
N-[3-(3-Pyridyl)propyl]trimethylendiamin,
N-(4-Pyridylmethyl)trimethylendiamin,
N-[1-(4-Pyridyl)ethyl]trimethylendiamin,
N-[1-(3-Pyridyl)ethyl]ethylendiamin,
N-[1-(4-Pyridyl)ethyl]ethylendiamin und
N-(4-Pyridylmethyl)tetramethylendiamin.

Zu speziellen Beispielen für die Ausgangsverbindungen der allgemeinen Formel (III) zählen

1-Nitro-2,2-bis(methylthio)ethylen,
1-Nitro-2,2-bis(ethylthio)ethylen und
2-Nitromethylen-1,3-dithiolan.

Das vorgenannte Verfahren wird durch das folgende typische Beispiel im einzelnen beschrieben.

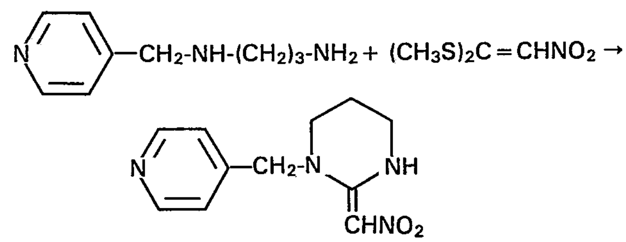

Zweckmässig kann das vorstehende Verfahren zur Herstellung der Verbindungen gemäss der vorliegenden Erfindung in einem Lösungsmittel oder Verdünnungsmittel durchgeführt werden. Zu diesem Zweck können sämtliche inerten Lösungsmittel oder Verdünnungsmittel verwendet werden.

Beispiele für solche Lösungsmittel oder Verdünnungsmittel umfassen Wasser; aliphatische, alicyclische und aromatische Kohlenwasserstoffe (die gegebenenfalls chloriert sein können), wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Methylisobutylketon, Nitrile wie Acetonitril, Propionitril und Acrylnitril, Alkohole wie Methanol, Ethanol, Isopropanol, Butanol und Ethylenglycol, Ester wie Ethylacetat und Amylacetat, Säureamide wie Dimethylformamid und Dimethylacetatamid, Sulfone und Sulfoxide wie Dimethylsulfoxid und Sulfolan sowie Basen wie Pyridin.

Das obige Verfahren kann innerhalb eines breiten Temperaturbereichs durchgeführt werden. Im allgemeinen kann es bei einer Temperatur zwischen −20°C und dem Siedepunkt der Mischung, zweckmässigerweise bei einer Temperatur zwischen etwa 0°C und etwa 100°C, durchgeführt werden. Zweckmässigerweise wird die Reaktion unter normalem Atmosphärendruck durchgeführt, jedoch ist es ebenfalls möglich, bei erhöhtem oder vermindertem Druck zu arbeiten.

Die Verbindungen der allgemeinen Formel (I) gemäss der vorliegenden Erfindung können auch mit Hilfe eines anderen, im folgenden schematisch dargestellten Verfahrens ii) hergestellt werden.

*Verfahren ii) (Alternativ-Verfahren)*

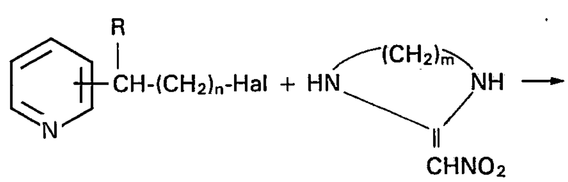

(IV) (V)

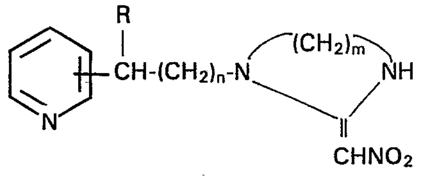

(I)

(In den vorstehenden Formeln haben R, m und n die im vorstehenden angegebene Bedeutung, und Hal bezeichnet ein Halogen-Atom.)

In dem durch das obige Reaktionsschema dargestellten Verfahren zur Herstellung der Verbindung der allgemeinen Formel (I) zählen zu speziellen Beispielen für die Verbindungen der allgemeinen Formel (IV), die ein Ausgangsstoff ist, 3-Pyridylmethylchlorid, 4-Pyridylmethylchlorid und 2-Pyridylmethylchlorid. Die entsprechenden Bromide sind ebenfalls zu nennen. Zu speziellen Beispielen für die Verbindung der allgemeinen Formel (V), die in gleicher Weise ein Ausgangsstoff ist, gehören 2-Nitromethylen-imidazolidin, 2-Nitromethylen-tetrahydropyrimidin und 2-Nitromethylen-hexahydro-1,3-diazepin.

Das vorgenannte Verfahren ii) wird durch das folgende Bezugsbeispiel im einzelnen beschrieben:

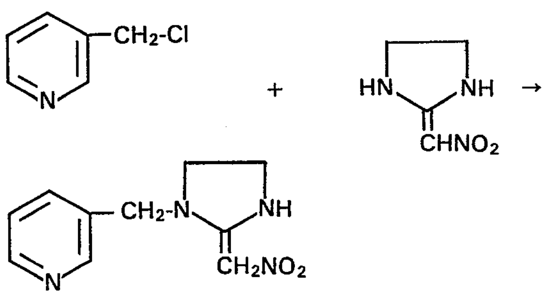

Das vorstehende Verfahren kann unter Verwendung der gleichen Lösungsmittel oder Verdünnungsmittel durchgeführt werden, wie sie im vorstehenden beispielhaft für das Verfahren i) aufgeführt sind.

Die vorstehende Reaktion kann in Gegenwart eines säurebindenden Mittels durchgeführt werden. Beispiele für das säurebindende Mittel sind die Hydroxide, Carbonate, Hydrogencarbonate und Alkoholate von Alkalimetallen sowie tertiäre Amine wie Triethylamin, Diethylanilin und Pyridin, die im allgemeinen verwendet werden.

Das vorstehende Verfahren kann wie im Fall des Verfahrens i) innerhalb eines weiten Temperaturbereiches durchgeführt werden. Die Reaktion wird zweckmässigerweise unter normalem Atmosphärendruck durchgeführt, jedoch ist es ebenfalls möglich, bei erhöhtem oder vermindertem Druck zu arbeiten.

Die Verbindungen der vorliegenden Erfindung können in Form ihrer Salze vorliegen. Die Salze können beispielsweise anorganische Salze, Sulfonate, organische Säure-Salze und Metall-Salze sein. Zu speziellen Beispielen für Verbindungen der vorliegenden Erfindung in Salz-Form zählen

1-(3-Pyridylmethyl)-2-nitromethylen)tetrahydropyrimidin-hydrochlorid,

1-(4-Pyridylmethyl)-2-nitromethylen)tetrahydropyrimidin-hydrochlorid,

1-(4-Pyridylmethyl)-2-nitromethylen)imidazolidin-hydrochlorid,

1-(3-Pyridylmethyl)-2-nitromethylen)imidazolidin-hydrochlorid,

1-(4-Pyridylmethyl)-2-nitromethylen)tetrahydropyrimidin-p-toluolsulfonat,

1-(4-Pyridylmethyl)-2-nitromethylen)tetrahydropyrimidin-succinat und

1-(4-Pyridylmethyl)-2-nitromethylen)tetrahydropyrimidin-kupfer(II)-acetat.

Als insektizide, mitizide und nematizide Mittel können die Verbindungen der vorliegenden Erfindung unmittelbar nach dem Verdünnen mit Wasser oder aber in Form verschiedenartiger Formulierungen zur Anwendung gebracht werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Gebrauch werden diese verschiedenen Formulierungen entweder unmittelbar oder nach dem Verdünnen mit Wasser auf die gewünschte Konzentration eingesetzt.

Zu den hier erwähnten landwirtschaftlich unbedenklichen Hilfsstoffen zählen beispielsweise Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe [z.B. n-Hexane, Petrolether, Erdöl-Fraktionen (z.B. Paraffinwachse, Kerosin, Leichtöle, Mittelöle und Schweröle), Benzol, Toluol und Xylol], halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methanol, Ethanol, Propanol und Ethylenglycol), Ether (z.B. Diethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alylsulfonsäureester (z.B. Natriumlaurylsulfat), Arylsulfonsäuren (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchloride) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z.B. Trichlorofluoromethan, Dichlorofluoromethan, 1,2,2-Trichloro-1,1,2-trifluoroethan, Chlorbenzol, verflüssigtes Erdgas (LNG) und niedere Ether), die Verbrennung steuernde Mittel für Räuchermittel (z.B. Nitrite, Zink-Pulver und Dicyandiamid), sauerstoff-abgebende Mittel (z.B. Chlorate), wirkungsverlängernde Mittel, Dispersions-Stabilisatoren [z.B. Casein, Tragant, Carboxymethylcellulose (CMC) und Polyvinylalkohol (PVA)] und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Erläuternde Beispiele für solche Anwendungsformen sind emulgierbare Konzentrate, Öl-Präparate, benetzbare Pulver, lösliche Pulver, Suspensionen, Stäubemittel, Granulate, pulvrige Präparate, Räuchermittel, Tabletten, Aerosole, Pasten und Kapseln.

Die insektiziden, mitiziden und nematiziden Mittel gemäss der vorliegenden Erfindung können etwa 0,1 bis etwa 95 Gew.-%, vorzugsweise etwa 0,5 bis etwa 90 Gew.-% des vorerwähnten Wirkstoffs enthalten.

Für den praktischen Gebrauch beträgt die geeignete Menge der aktiven Verbindung in den vorgenannten verschiedenartigen Formulierungen und gebrauchsfertigen Präparaten im allgemeinen etwa 0,0001 bis etwa 20 Gew.-%, vorzugsweise etwa 0,005 bis etwa 10 Gew.-%.

Der Gehalt des Wirkstoffs kann in geeigneter Weise je nach der Art der Formulierung, dem Verfahren, Zweck, der Zeit und dem Ort seiner Anwendung sowie dem Zustand des Auftretens der zu bekämpfenden schädlichen Insekten, Milben oder Zecken und Nematoden variiert werden.

Erforderlichenfalls können die Verbindungen gemäss der vorliegenden Erfindung weiterhin auch in Kombination mit anderen Agrochemikalien verwendet werden, beispielsweise mit anderen Insektiziden, Fungiziden, anderen Mitiziden, anderen Nematiziden, Anti-Virus-Mitteln, Herbiziden, Pflanzen-Wachstrumregulatoren und Lockstoffen [z.B. Organophosphat-Verbindungen, Carbamat-Verbindungen, Dithio(oder thiol)carbamat-Verbindungen, Organochlor-Verbindungen Dinitro-Verbindungen, organischen Schwefel- oder metallorganischen Verbindungen, Antibiotika, substituierten Diphenylether-Verbindungen, Harnstoff-Verbindungen und Triazin-Verbindungen] und/oder Düngemitteln.

Den im vorstehenden bezeichneten erfindungsgemässen Wirkstoff enthaltende verschiedenartige Mittel und gebrauchsfertige Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Giessen usw.), Räuchern, Bodenbehandlung (Vermischen mit dem Boden, Spritzen, Bedampfen, Giessen usw.), Oberflächen-Anwendung (z.B. Beschichten, Aufbringen in Form von Bändern, Pulverbeschichten, Bedecken usw.), Eintauchen und Ködern. Sie können auch mittels des sogenannten Ultra-Low-Volume-Sprühverfahrens aufgebracht werden. Nach diesem Verfahren kann der Wirkstoff sogar in einer Konzentration von 100% eingearbeitet sein.

Die Aufwandmenge pro Flächeneinheit beträgt beispielsweise etwa 0,03 bis etwa 10 kg/ha, vorzugsweise etwa 0,3 bis etwa 6 kg/ha. In besonders gelagerten Fällen können oder sollten sogar die Aufwandmengen ausserhalb des angegebenen Bereichs liegen.

Gemäss der vorliegenden Erfindung kann ein insektizides, mitizides und nematizides Mittel zur Verfügung gestellt werden, das als Wirkstoff die Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel usw. enthält.

Die vorliegende Erfindung macht weiterhin ein Verfahren zur Bekämpfung von Insekten, Milben oder Zecken und Nematoden verfügbar, das darin besteht, dass eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel usw. auf schädliche Insekten, Milben oder Zecken und Nematoden und/oder deren Lebensraum oder den Ort ihres Auftretens aufgebracht wird.

Die vorliegende Erfindung wird durch die folgenden Beispiele im einzelnen erläutert, ist jedoch nicht auf diese speziellen Beispiele allein beschränkt.

*Beispiel 1*

Eine Mischung aus N-(4-Pyridylmethyl)trimethylendiamin (16,5 g), 1-Nitro-2,2-bis(methylthio)ethylen (16,5 g) und Methanol (100 ml) wurde 2 h unter Rühren zum Rückfluss erhitzt. Das erzeugte Gas wurde in einer Alkali-Falle aufgefangen. Die Reaktionsmischung wurde auf Raumtemperatur gekühlt, und die erhaltenen Kristalle wurden abfiltriert, wonach 1-(4-Pyridylmethyl)-2-(nitromethylen)tetrahydropyrimidin (16 g) der nachstehenden Formel in Form blassgelber Kristalle erhalten wurde. Einengen

des Filtrats lieferte weitere 3 g dieser Verbindung. Schmelzpunkt: 226-228 °C.

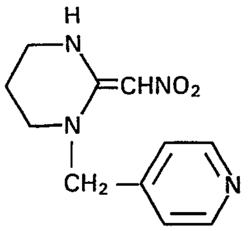

(Verbindung Nr. 1)

*Beispiel 2*

Eine Mischung aus N-(3-Pyridylmethyl)ethylendiamin (15,1 g), 1-Nitro-2,2-bis(methylthio)ethylen (18,2 g) und Benzol (150 ml) wurde 4 h unter Rühren zum Rückfluss erhitzt. Das erzeugte Gas wurde in einer Alkali-Falla aufgefangen. Die Reaktionsmischung wurde auf Raumtemperatur gekühlt und zum Sammeln der Kristalle filtriert. Umkristallisieren aus Methanol lieferte 1-(3-Pyridylmethyl)-2-(nitromethylen)imidazolidin (17 g) der nachstehenden Formel in Form blassgelber Kristalle. Schmelzpunkt: 169 bis 171 °C.

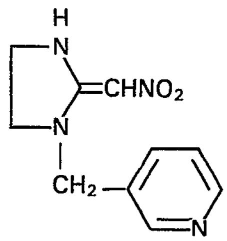

(Verbindung Nr. 2)

Die nachstehende Tabelle 1 führt die Verbindungen der vorliegenden Erfindung auf, die weitgehend in gleicher Weise wie in den Beispielen 1 und 2 synthetisiert werden.

TABELLE 1

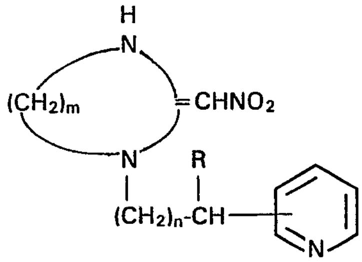

| Verbindung Nr. | R | m | n | Pyridin-Bindungs-Position | Physikalische Konstante: Schmelzpunkt |
|---|---|---|---|---|---|
| 3 | H | 2 | 0 | 2- | 209-212 °C |
| 4 | H | 2 | 0 | 4- | 203-206 °C |
| 5 | H | 2 | 1 | 4- | 219-221 °C |
| 6 | H | 3 | 0 | 2- | 202-203 °C |
| 7 | H | 3 | 1 | 2- | 105-108 °C |
| 8 | H | 3 | 0 | 3- | 207-209 °C |
| 9 | $-CH_3$ | 3 | 0 | 3- | 171-172 °C |
| 10 | H | 3 | 2 | 3- | 137-140 °C |
| 11 | $-CH_3$ | 3 | 0 | 4- | 222-225 °C |
| 12 | $-CH_3$ | 2 | 0 | 3- | 121-122 °C |
| 13 | $-CH_3$ | 2 | 0 | 4- | 189-190 °C |
| 14 | H | 4 | 0 | 4- | |
| 15 | $-C_2H_5$ | 2 | 0 | 4- | |
| 16 | $-C_3H_7$-iso | 3 | 0 | 3- | |
| 17 | $-C_3H_7$-iso | 3 | 0 | 4- | |
| 18 | $CH_3$ | 2 | 1 | 3- | |
| 19 | $-C_2H_5$ | 2 | 0 | 3- | |

Spezielle Beispiele für Salze der oben aufgeführten Verbindungen sind nachstehend aufgezeigt.

*Beispiel 3*

1-(4-Pyridylmethyl)-2-(nitromethylen)tetrahydropyrimidin (0,74 g), das in Beispiel 1 erhalten worden war, wurde in Ethanol (20 ml) gelöst, und konzentrierte Salzsäure (2 ml) wurde zugegeben. Die Reaktionsmischung wurde 24 h bei Raumtemperatur gerührt. Die ausgefallenen Kristalle wurden durch Filtration gesammelt und vollständig mit Ether gewaschen, wonach das Hydrochlorid (0,72 g) der Verbindung 1 der vorliegenden Erfindung erhalten wurde; Schmelzpunkt: 185°C (Zers.).

*Beispiel 4*

Die in Beispiel 1 erhaltene Verbindung Nr. 1 (0,73 g) wurde in einem Mischlösungsmittel aus Aceton (10 ml) und Chloroform (50 ml) gelöst, und p-Toluolsulfonsäurehydrat (0,63 g) wurde zugesetzt. Die Mischung wurde 2 h kräftig geschüttelt und dann 3 h bei Raumtemperatur stehen gelassen. Die ausgefallenen Kristalle wurden durch Filtration gesammelt und vollständig mit Aceton gewaschen, wonach das p-Toluolsulfonat (1,2 g) der Verbindung 1 der vorliegenden Erfindung erhalten wurde; Schmelzpunkt: 170-176°C.

*Beispiel 5*

Die in Beispiel 1 erhaltene Verbindung Nr. 1 (0,74 g) wurde in wasserfreiem Ethanol (50 ml) gelöst, und Kupfer(II)-acetat (0,665 g) wurde zugesetzt. Die Mischung wurde etwa 24 h bei Raumtemperatur aufbewahrt und dann filtriert. Der erhaltene blassgrüne Feststoff wurde mit einer kleinen Menge Wasser und danach gut mit Chloroform gewaschen und schliesslich im Vakuum getrocknet, wonach das Kupfer-Salz (1,15 g) der Verbindung 1 der vorliegenden Erfindung erhalten wurde; Schmelzpunkt: 220-225°C (Zers.).

Tabelle 2 zeigt die oben genannten Salze der Verbindung der vorliegenden Erfindung und ein weiteres Beispiel für ein Salz (Salz mit einer organischen Säure).

TABELLE 2

| Verbindung Nr. | Formel | Physikalische Konstante |
|---|---|---|
| 1-a | | |
| 1-a | 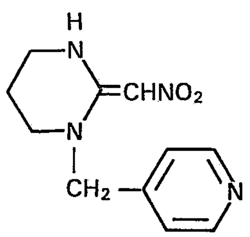 · HCl | beschrieben in Beispiel 3 |
| 1-b | 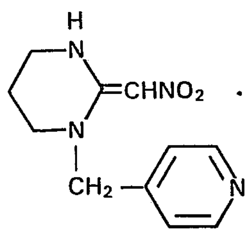 · 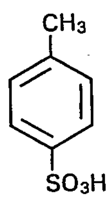 | beschrieben in Beispiel 4 |
| 1-c | 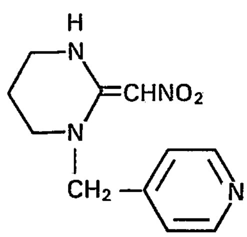 · 1/2 Cu(OCOCH$_3$) | beschrieben in Beispiel 5 |
| 1-d | 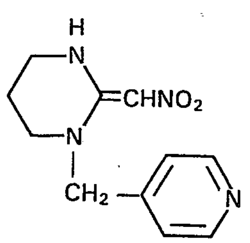 · 1/2 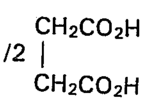 | Schmelzpunkt: 180-185°C |

*Beispiel 6 (Benetzbares Pulver)*

15 Teile der Verbindung Nr. 4 der Erfindung, 80 Teile eines Gemisches (1:5) aus pulvriger Diatomeenerde und Tonpulver, 2 Teile Natriumalkylbenzolsulfonat und 3 Teile Natriumalkylnapthalinsulfonat/Formaldehyd-Kondensat werden pulverisiert und zu einem benetzbaren Pulver vermischt. Dieses wird mit Wasser verdünnt und auf schädliche Insekten, Milben oder Nematoden und/oder ihren Lebensraum oder den Ort ihres Auftretens aufgesprüht.

*Beispiel 7 (Emulgierbares Konzentrat)*

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf schädliche Insekten, Milben oder Nematoden und/oder ihren Lebensraum oder den Ort ihres Auftretens aufgesprüht.

*Beispiel 8 (Stäubemittel)*

2 Teile der Verbindung Nr. 1 der Erfindung und 98 Teile Tonpulver werden pulverisiert und gemischt, wodurch ein Stäubemittel hergestellt wird. Dieses wird über schädlichen Insekten, Milben oder Nematonen und/oder ihrem Lebensraum oder dem Ort ihres Auftretens ausgestreut.

*Beispiel 9 (Granulat)*

Wasser (25 Teile) wird zu einer Mischung aus 10 Teilen der Verbindung Nr. 8 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngrösse von 0,43 bis 2,0 mm (10 bis 40 mesh) verarbeitet, das dann bei 40°C bis 50°C getrocknet wird, wodurch ein Granulat gebildet wird. Das Granulat wird über schädlichen Insekten, Milben oder Nematoden und/oder ihrem Lebensraum oder dem Ort ihres Auftretens ausgestreut.

*Beispiel 10 (Granulat)*

Ein Drehmischer wird mit 95 Teilen Tonmineral-Teilchen mit einer Teilchengrössen-Verteilung zwischen 0,2 und 2 mm beschickt, und unter Drehen des Mischers werden 5 Teile der Verbindung Nr. 1 der Erfindung auf die Tonmineral-Teilchen zur gleichmässigen Benetzung derselben aufgesprüht. Die feuchte Mischung wird bei 40°C bis 50°C getrocknet, wodurch ein Granulat gebildet wird. Das Granulat wird über schädliche Insekten, Milben oder Nematoden und/oder ihrem Lebensraum oder dem Ort ihres Auftretens ausgestreut.

*Beispiel 11 (Öl-Präparat)*

Die Verbindung Nr. 1 der Erfindung (0,5 Teile) und 99,5 Teile Kerosin werten unter Rühren miteinander vermischt, wodurch ein Öl-Präparat gebildet wird. Dieses wird auf schädliche Insekten, Milben oder Nematoden und/oder ihren Lebensraum oder den Ort ihres Auftretens aufgesprüht.

*Beispiel 12 (Biologischer Test)*

*Test mit gegen Organophosphor-Mittel resistenten Nephotettix cincticeps:*

*Herstellung eines Test-Präparats:*

| | | |
|---|---|---|
| Lösungsmittel: | Xylol | 3 Gew.-Teile |
| Emulgator: | Polyoxyethylen-alkylphenylether | 1 Gew.-Teil |

Zur Herstelung eines geeigneten Präparats wurde 1 Gew.-Teil der aktiven Verbindung mit der oben bezeichneten Menge Lösungsmittel, das die oben angegebene Menge Emulgator enthielt, vermischt. Die Mischung wurde mit Wasser auf eine vorher festgesetzte Konzentration verdünnt.

*Test-Verfahren:*

Auf Reispflanzen von etwa 10 cm Höhe, die jeweils in Töpfe von 12 cm Durchmesser gepflanzt waren, wurden pro Topf 10 ml der mit Wasser verdünnten, eine vorbestimmte Wirkstoff-Konzentration aufweisenden Lösung jeder der wirksamen Verbindungen, die wie oben angegeben hergestellt wurde, gesprüht. Die aufgesprühte Chemikalie wurde trocknen gelassen, und über die Reispflanzen wurde ein Drahtkorb von 7 cm Durchmesser und 14 cm Höhe gestülpt, unter dem 30 ausgewachsene weibliche Exemplare von Nephotettix cincticeps, die gegen Organophosphor-Mittel resistent waren, ausgesetzt wurden. Die Töpfe wurden jeweils in einem Raum mit konstanter Temperatur aufbewahrt, und 2 Tage später wurde die Zahl der toten Insekten bestimmt und das Vernichtungsverhältnis berechnet.

Gegenüber den Vergleichs-Verbindungen mit den Formeln (A-1) bzw. (B-1)

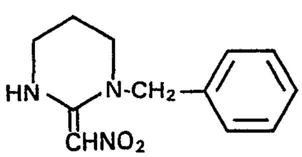

(A-1)

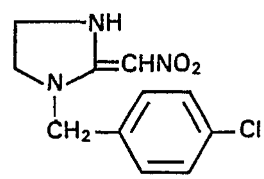

(B-1)

zeigten z.B. die nachstehenden Verbindungen gemäss der vorliegenden Erfindung unerwartete Vorteile: Verbindung Nr. 1, 1-a, 2 und 4.

*Beispiel 13 (Biologischer Test)*

*Test mit Laternenträgern:*

*Test-Verfahren:*

Eine wie in Beispiel 12 hergestellte wässrige Verdünnung mit vorher festgelegter Konzentration der aktiven Verbindung wurde auf etwa 10 cm hohe Reispflanzen, die in Töpfen mit einem Durchmesser von 12 cm gezogen wurden, in einer Menge von 10 ml pro Topf aufgesprüht. Die aufgesprühte Chemikalie wurde trocknen gelassen, und über die Reispflanzen wurde ein Drahtkorb von 7 cm Durchmesser und 14 cm Höhe gestülpt. 30 ausgewachsene weibliche Exemplare von Nilaparvata lugens Stal. eines Stammes, der Resistenz gegen Organophosphor-Mittel zeigte, wurden unter dem Drahtkorb ausgesetzt. Die

Töpfe wurden in einem Raum mit konstanter Temperatur aufbewahrt, und 2 Tage später wurde die Zahl der toten Insekten bestimmt. Das Vernichtungsverhältnis wurde danach berechnet.

In gleicher Weise wurde das Vernichtungsverhältnis an Sogatella furcifera Horvath und Organophosphor-resistenten Loadelphax striatella Fallen berechnet.

Gegenüber den Vergleichs-Verbindungen mit den Formeln (A-1) bzw. (B-1) zeigten z.B. die nachstehenden Verbindungen gemäss der vorliegenden Erfindung unerwartete Vorteile: Verbindung Nr. 1, 1-a, 1-b, 1-d, 2, 4, 6, 8 und 9.

*Beispiel 14 (Biologischer Test)*

*Test mit gegen Organophosphor-Chemikalien und Carbamat-Chemikalien resistenten Myzodes persicae (grünen Pfirsichblattläusen):*

*Test-Verfahren:*

Gezüchtete grüne Pfirsichblattläuse, die Resistenz gegen Organophosphor-Chemikalien und Carbamat-Chemikalien zeigten, wurden auf Setzlingen von Eierfrüchten (schwarzen länglichen Auberginen) von etwa 20 cm Höhe ausgesetzt, die in unglasierten Töpfen mit einem Durchmesser von 15 cm gezogen worden waren (etwa 200 Blattläuse pro Setzling). Einen Tag nach dem Aussetzen wurde eine wie in Beispiel 12 hergestellte wässrige Verdünnung jeder der aktiven Verbindungen mit einer vorher festgelegten Konzentration in genügender Menge mit Hilfe einer Spritzpistole auf die Pflanzen aufgesprüht. Nach dem Sprühen wurden die Töpfe in einem Gewächshaus bei 28°C stehen gelassen. 24 Stunden nach dem Sprühen wurde das Vernichtungsverhältnis berechnet. Für jede Verbindung wurde der Test als Doppelbestimmung durchgeführt.

Gegenüber den Vergleichs-Verbindungen mit den Formeln (A-1) bzw. (B-1) und Estox (Handelsprodukt) zeigten z.B. die nachstehenden Verbindungen gemäss der vorliegenden Erfindung unerwartete Vorteile: Verbindung Nr. 1, 2 und 8.

**Patentansprüche**

1. Nitromethylen-Derivate der allgemeinen Formel

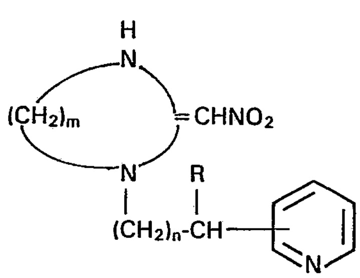

(I)

in der

R ein Wasserstoff-Atom, Methyl, Ethyl, Propyl, i-Propyl, n-Butyl, i-Butyl, sec.Butyl oder tert.Butyl bezeichnet,

m für 2, 3 oder 4 steht und

n für 0, 1, 2 oder 3 steht,

oder ein Salz derselben.

2. Nitromethylen-Derivate oder ein Salz desselben nach Anspruch 1, dadurch gekennzeichnet, dass m 2 oder 3 ist.

3. 1-(3-Pyridylmethyl)-2-nitromethylen)imidazolin nach Anspruch 1 oder 2, gekennzeichnet durch die Formel

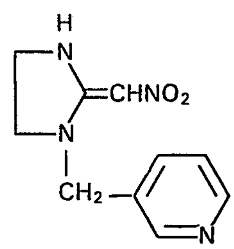

4. 1-(4-Pyridylmethyl)-2-nitromethylen)imidazolin nach Anspruch 1 oder 2, gekennzeichnet durch die Formel

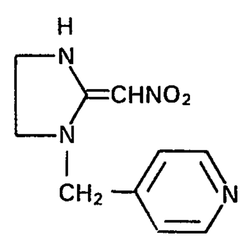

5. 1-(3-Pyridylmethyl)-2-nitromethylen)tetrahydropyrimidin nach Anspruch 1 oder 2, gekennzeichnet durch die Formel

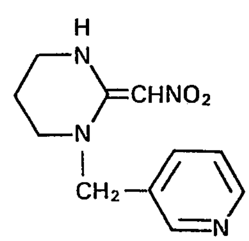

6. 1-(4-Pyridylmethyl)-2-nitromethylen)tetrahydropyrimidin nach Anspruch 1 oder 2, gekennzeichnet durch die Formel

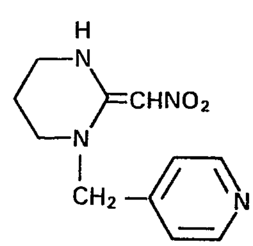

7. Verfahren zur Herstellung eines Nitromethylen-Derivats der allgemeinen Formel

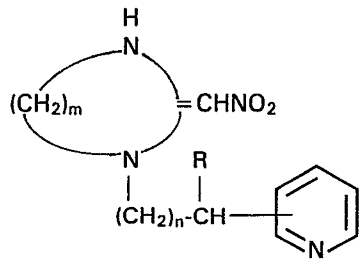

(I)

in der

R ein Wasserstoff-Atom, Methyl, Ethyl, Propyl, i-Propyl, n-Butyl, i-Butyl, sec.Butyl oder tert.Butyl bezeichnet,

m für 2, 3 oder 4 steht und

n für 0, 1, 2 oder 3 steht,

oder eines Salzes desselben, dadurch gekennzeichnet dass eine Verbindung der allgemeinen Formel

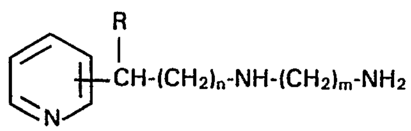

(II),

in der R, m und n die im vorstehenden angegebenen Bedeutungen haben, mit einer Verbindung der allgemeinen Formel

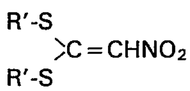

(III),

in der jedes R' eine niedere Alkyl-Gruppe bezeichnet oder die beiden R'-Gruppen zusammen eine niedere Alkylen-Gruppe mit wenigstens 2 Kohlenstoff-Atomen bezeichnen und mit den ihnen benachbarten Schwefel-Atomen einen Ring bilden können, umgesetzt wird.

8. Insektizides, mitizides und nematizides Mittel, enthaltend als Wirkstoff ein Nitromethylen-Derivat der allgemeinen Formel (I) gemäss Anspruch 1 oder ein Salz desselben.

## Claims

1. Nitromethylene derivatives of the general formula

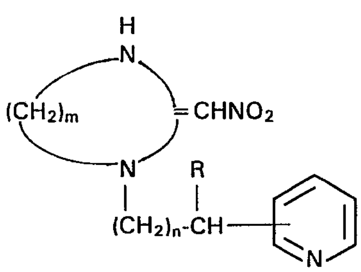

(I)

in which

R designates a hydrogen atom, methyl, ethyl, propyl, i-propyl, n-butyl, i-butyl, sec.butyl or tert.butyl,

m represents 2, 3 or 4 and

n represents 0, 1, 2 or 3,

or a salt thereof.

2. Nitromethylene derivatives or a salt thereof according to Claim 1, characterised in that m is 2 or 3.

3. 1-(3-Pyridylmethyl)-2-nitromethylene)imidazoline according to Claim 1 or 2, characterised by the formula

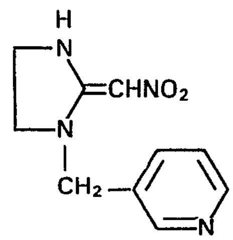

4. 1-(4-Pyridylmethyl)-2-nitromethylene)imidazoline according to Claim 1 or 2, characterised by the formula

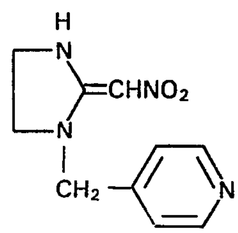

5. 1-(3-Pyridylmethyl)-2-nitromethylene)tetrahydropyrimidine according to Claim 1 or 2, characterised by the formula

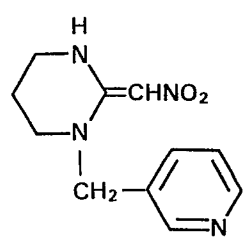

6. 1-(4-Pyridylmethyl)-2-nitromethylene)tetrahydropyrimidine according to Claim 1 or 2, characterised by the formula

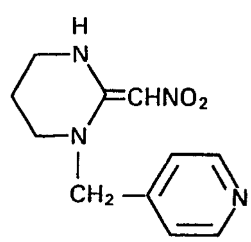

7. Process for the preparation of a nitromethylene derivative of the general formula

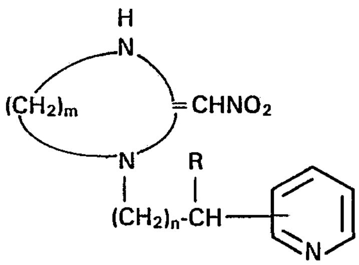

(I)

in which

R designates a hydrogen atom, methyl, ethyl, propyl, i-propyl, n-butyl, i-butyl, sec.butyl or tert.butyl,

m represents 2, 3 or 4 and

n represents 0, 1, 2 or 3,

or a salt thereof, characterised in that a compound of the general formula

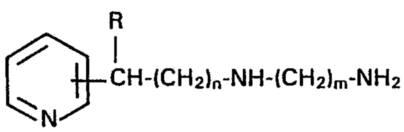

(II)

in which

R, m and n have the meanings given above, is reacted with a compound of the general formula

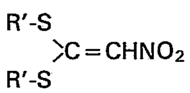

(III),

in which each R' designates a lower alkyl group or the two R' groups together designate a lower alkylene group with at least 2 carbon atoms and can form a ring with the sulphur atoms adjacent to them.

8. Insecticidal, miticidal and nematicidal agent, containing a nitromethylene derivative of the general formula (I) according to Claim 1 or a salt thereof as the active compound.

**Revendications**

1. Dérivé nitrométhylénique de formule générale

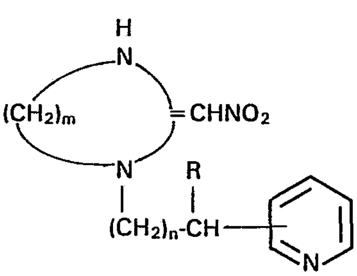

(I)

dans laquelle

R est un atome d'hydrogène, un groupe méthyle, éthyle, propyle, isopropyle, n-butyle, isobutyle, sec.-butyle ou tertio-butyle,

m a la valeur 2, 3 ou 4 et

n a la valeur 0, 1, 2 ou 3,

ou un sel de ce dérivé.

2. Dérivé nitrométhylénique ou un sel de ce dérivé suivant la revendication 1, caractérisé en ce que m a la valeur 2 ou 3.

3. 1-(3-pyridylméthyl)-2-nitrométhylène)imidazoline suivant la revendication 1 ou 2, caractérisé par la formule

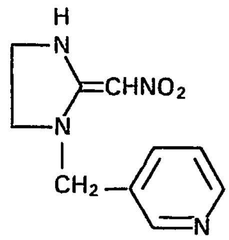

4. 1-(4-pyridylméthyl)-2-nitrométhylène)imidazoline suivant la revendication 1 ou 2, caractérisé par la formule

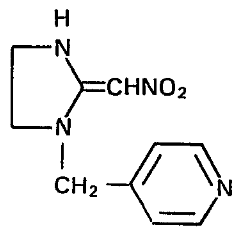

5. 1-(3-pyridylméthyl)-2-nitrométhylène)tétrahydropyrimidine suivant la revendication 1 ou 2, caractérisé par la formule

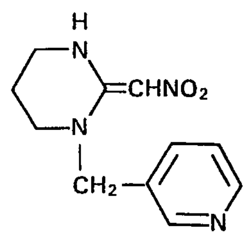

6. 1-(4-pyridylméthyl)-2-nitrométhylène)tetrahydropyrimidine suivant la revendication 1 ou 2, caractérisé par la formule

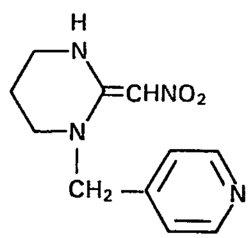

7. Procédé de production d'un dérivé nitrométhylénique de formule générale

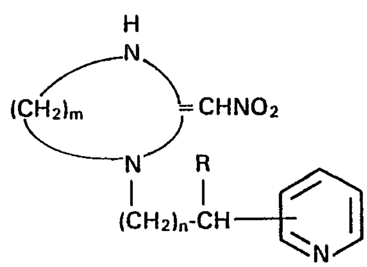

(I),

dans laquelle

R est un atome d'hydrogène, un groupe méthyle, éthyle, propyle, isopropyle, n-butyle, isobutyle, sec.-butyle ou tertio-butyle,

m a la valeur 2, 3 ou 4 et

n a la valeur 0, 1, 2 ou 3,

ou un sel de ce dérivé, caractérisé en ce qu'on fait reagier un composé de formule générale

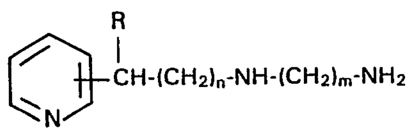

(II)

dans laquelle R, m et n ont les définitions indiquées ci-dessus, avec un composé de formule générale

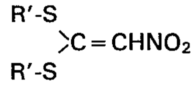

(III),

dans laquelle chaque R' représente un groupe alkyle inférieur ou bien les deux groupes R' forment conjointement un groupe alkylène inférieur ayant au moins deux atomes de carbone et peuvent former un noyau avec les atomes de soufre qui leur sont adjacents.

8. Composition insecticide, acaricide et nématicide, contenant comme substance active un dérivé nitrométhylénique de formule générale (I) suivant la revendication 1 ou un sel de ce dérivé.